# EUROPEAN PATENT SPECIFICATION

(11) **EP 0 392 315 B1**
(45) Date of publication and mention of the grant of the patent: **21.09.1994**
(21) Application number: 90106311.5
(22) Date of filing: 02.04.1990
(51) Int. Cl.: A61K 37/24, A61K 37/02

(54) **Pure Histone H1 for use in therapeutic procedures**
Reines Histon H1 zur therapeutischen Verwendung
Histone H1 pure utilisable dans des procédés thérapeutiques

(30) Priority: 03.04.1989 US 332658
(43) Date of publication of application: 17.10.1990
(73) Proprietor: SYMBIOTEC Gesellschaft zur Forschung und Entwicklung auf dem Gebiet der Biotechnologie GmbH, D-35745 Herborn (DE)
(72) Inventor: Zeppezauer, Michael, Prof.-Dr., D-6601 Saarbrücken-Scheidt (DE); Reichhart, Robert, Dr., D-6650 Homburg/Saale (DE)
(74) Representative: Pätzold, Herbert, Dr.-Ing.

(56) References cited:
- EP-A- 0 149 468
- DE-A- 3 737 274
- PROC. NATL. ACAD. SCI. USA, vol. 82, no. 15, August 1985, pages 4871-4875, Washington, DC, US; R. REICHHART et al.: "Preparations of homeostatic thymus hormone consist predominantly of histones 2A and 2B and suggest additional histone functions"
- BIOLOGICAL ABSTRACTS, vol. 75, no. 1, 1983, page 339, abstract no. 3258, Biological Abstracts, Inc., Philadelphia, PA, US; V.G. MOROZOV et al.: "Isolation, purification and identification of an immunodulating polypeptide from human and calf thymus", && BIOKHIMIYA 46(9): 1652-1659, 1981
- CHEMICAL ABSTRACTS, vol. 110, no. 7, 13th February 1989, page 533, abstract 55827v, Columbus, Ohio, US; & JP-A-63 139 134 (KYOWA HAKKO KOGYO CO., LTD) 10-06-1988

## Description

According to the present state of knowledge histones H1, H2A, H2B, H3 and H4 are the essential components to the cell nucleic and are together with DNA the substantial components of chromatin.

In the European Patent 0 149 468 it is proposed that pure histone H2A and/or H2B or H3 or active segments thereof (the evolutionary variable part or partial sequences of this part or the N-terminal part). It is nothing said about histone H1 and the histone dimer H2A:H2B. The histones have hormonal or hormone-like and immuno-stimulating functions. Such functions so far have never been brought in connection with histones. For the experts it was novel and surprising that the histones are freely moving in the blood circulation and the lymphatic system of healthy organisms and will bring about a biological response due to the interaction with specific receptors of living cells. There is provided the use of histone for stimulating the immune system, for the therapy of the immune system, for the treatments of immune deficiency, of the consequences of the thymectomy and the following of massive irradiation of the thymus, for the damping of thyroxine, desoxycorticosterone, thyrotropin, gonadotropin and for the strength of growth hormone.

In U.S Patent 4 415 553 it is proposed to prepare an antigen from human or animal cancer cells, which is injected to persons or animals suffering from cancer in order to incite the production of antibodies, which are not only intended to destroy the antigens but also the very own cancer cells. From the cancer cells a malignant total histone is being isolated, which is not divided into the individual histones, and the total histone is bonded to malignant DNA and RNA in order to form the antigen.

In "Chemical Abstrats" 72, 109724 (1970) it was proposed to isolate fragments of the histones which are rich with arginine and lysine, to charge these fragments with reactive groups and to take advantage of their bondage to nucleic acids for chemically changing the nucleic acids by means of said reactive groups. Said literature discloses the idea of using specific histone fragments as a carrier for chemotherapeutic substances applied in the treatment of cancer.

In "Chemical Abstracts" 74, 85743 (1971) it is said that the resulting total histone fraction will prevent the formation of antibodies against T₂-bacteriophages if given simultaneously with the antigen (the phage) and furthermore in very large doses (50 mg/kg).

In "Chemical Abstracts" 73, 96837 (1970) the immuno-suppressive effect of total histone is described by the example of a human skin transplantation.

In Biological Abstracts 75(1983), Abstract No. 3258 and Biokhimiya, Vol. 46, No. 9, pp. 1652-1659 (1981) it was erroneously assumed that the thymus contains not severeal factors that affect difference stages of formation and differentiation of the T-lymphocytes but only on factor which would be thymarin with a molecular weight of about 2000-3000. In this connection it was further erroneously assumend that the only one immuno modulating factor thymarin would be similar to the histone H1 of the calf thymus so that histone H1 as one of the essential components of the cell nucleic also of the thymus would have an important role in the formation and differentiation of the T-lymphocytes. But this erroneously assumed similarity is not feasible because the known molecular weight of histone H1 of about 23.000 is about ten time higher than the molecular weigth of thymarin. Further it was not known that histone H1 is able to free moving in the serum. The assumed and not proved hormone like activity of histone H1 as one of the essential components of the cell nucleic is not comparable with the hormone like activity of free moving substances in the serum.

DE-A-37 37 274 discloses only the use of pure histones H2A and/or H2B as hormone or hormon like effective substances for use in the therapeutic procedures but it is nothing said about histone H1 including all subtyps or active segments thereof.

EP-A-0 149 468 discloses the use of at least one histone and/or at least one histone segment having hormonal activity for use in therapeutic and diagnostic procedures. In this connection the histones H2A, H2B and H3 are discussed only without saying anything about the other histones.

The above-mentioned state of art neither discloses nor suggested the discovery of the invention that pure histone H1 including all subtyps or active segments thereof may be used in therapeutic procedures.

It is the problem of the invention to provide histones or histon complexes other then H2A, H2B, H3 or specific therapeutic purposes and to provide the use of histones in new therapeutic procedures.

The problem is solved by using pure histone H1 including all subtyps or active segments thereof with hormone or hormone like activity especially thymic hormone like activity for use in the therapeutic procedures as for the immunotherapy; the therapy of endocrine malfunctions and cancer therapy (the treatment of melanoma, sarcoma, mesothelioma, malignant deceases, especially those of the lymphatic system originating from malignant B- and T-cells such as B-lymphoplastic lymphoma, myelomic leukemia, Burkitt lymphoma); the treatment of the consequences of the thymectomy or the following massive irradiation of the thymus; the treatment of radiation-induced leukemia or carcinoma and the treatment of AIDS-diseases; for suppressing the activity of the thyroxine or ACTH or desoxycorticosterone or thyrotropin or gonadotropin and for supporting the function of the hypophysis; the treatment of after-effects of thymectomy resulting from the removal of the thymus gland and the treatment with the purpose of the strengthing the activity of the growth hormone.

The pure histone H1 including all subtyps or active segments thereof is extracted from an endocrine gland of an animal especially from the gland of thymus gland of calf.

In the following examples the therapeutic efficiency of pure histone H1 including all subtyps or active segments thereof is shown:

### Preparation of H1 Histones

Thymus glands from calf were commercially obtained by slaughter houses. Glands taken directly from animals were immediately frozen on dry ice and stored at -30°C.

Whole glands were cut to pieces and homogenized in three volumes of 0,3 M sucrose containing 3 mM CaCl₂. The homogenate was centrifuged for 10 minutes at 200 g. The combined crude pellets were homogenized in 10 volumes of 2,5 M sucrose, 3 mM CaCl₂ and centrifuged for 60 min at 45 000 g. The pellets containing the nuclei were combined and washed twice with 0,5 M sucrose, 3 mM CaCl₂ and once with 0,15 M NaCl, 10 mM EDTA. After each step of washing the pellets were centrifuged at 1000 g for 10 min.

Total histones ( H1, H2A, H2B, H3, H4 ) were extracted from the combined nuclear pellets by treatment with 0,4 N H₂SO₄ at 0°C for 15 h. The mixture was centrifuged at 70 000 g for 30 min, 5 vol. of cooled ethanol was added to the supernatant, and the mixture was stood overnight at -20°C. H1 histones were extracted from the mixture with 5% perchloric acid for 3 h, and the mixture was centrifuged at 200 g for 10 min subsequently. The supernatant was dialyzed against 1 mM acetic acid overnight. H1 histone was precipitated by the addition of 100% trichloroacetic acid ( 1 gm/ml ) to a final concentration of 20% at 0°C. After 15 min the precipitate was collected by centrifugetion at 3 500 g for 15 min and washed once with acidified acetone ( 0,5 ml conc. HCl / 100 ml ) and twice with acetone. After centrifugation at 500 g for 10 min the pellets were soluted in 1 mM acetic acid and lyophilized to obtain H1 histones.

The action of Histone H1 was tested in vitro on six human cancer cell lines originating from B-cells of the lymphatic system. The concentration of H1 tested was 250 mcg/ml medium for the following cancers strains; Daudi, EB 2, CCRF SB, CCRF CEM and Namalwa and 180 mcg/ml for the IM9 strain. A maximal decay of cancer cells was observed between the 3^{rd} and 6^{th} day and reached up to 100%. 4 cancer cell lines began to grow again, but did not reach the initial cell concentration. The treatment of normal spleen cells of mice Showed that H1 had no significant toxicity on these cells at a concentration of 50-200 mcg/ml under the conditions employed.

The following cell lines has been used:
DAUDI: Burkitt Lymphoma of a 16 year old negro. Translocation of parts of the chromosomes 8 and 14 led to the activation of an oncogene. There are binding sites for the fc-receptor and for receptors for complement and immunoglobulins.

EB2: Burkitt Lymphoma of a 7 year old negro. The line contains free active Epstein-Barr virus particles.

IM9: Myelogenic Leucemia of a female patient. The cell line is able to produce IgG. There are receptors for human growth factors, insulin and calcitonin.

CCRF SB: B-lymphoblatic Lymphoma of a 11 year old caucasic girl. The line is not able to produce IgG.

CCRF CEM: B-lymphoblastic Lymphoma of a 4 year old caucasic boy. The line is able to produce IgG. No common receptors are B-cells on the membranes.

Namalwa: Burkitt Lymphoma
The tested cell lines were obtained from FLOW Lab. and grew in RPMI-1640 medium with 10% FCS in a humified incubator with 5,5% CO₂. 2x10⁵ cells/ml medium were incubated with 180- 250 mcg hormone/ml and spread in 96 well dishes. The viable cells were counted by the ethidium bromide and acridine orange method (EB/AO).

### Example 1

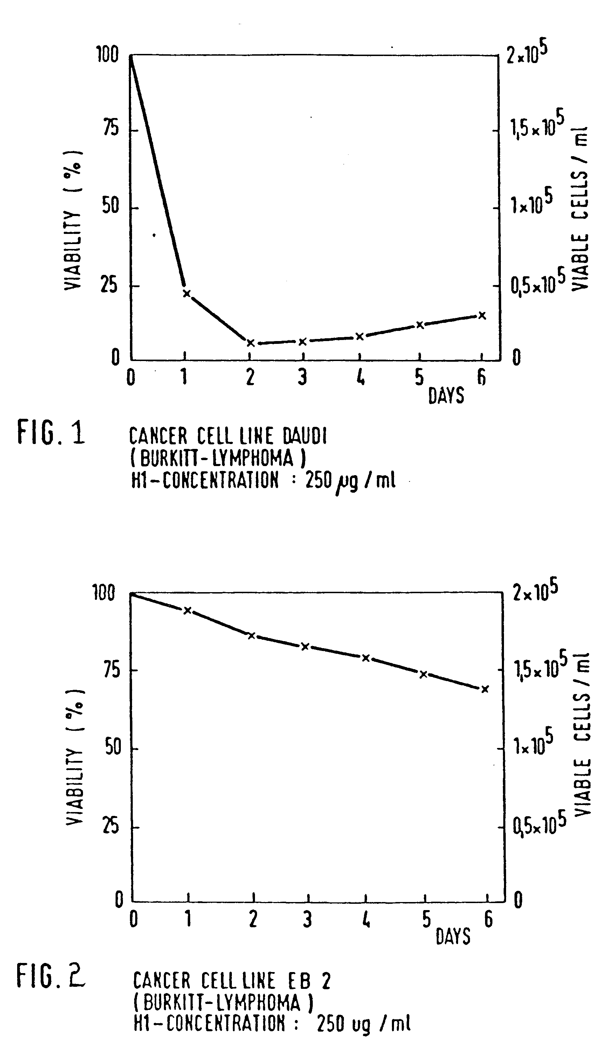

DAUDI showed a 96 % decay of cells after two days and had 20 % viable cells at the end of the experiment (Fig. 1).

### Example 2

EB2 showed a continous dying of cells up to the 6th day and had 70 % vital cells at last (Fig. 2).

### Example 3

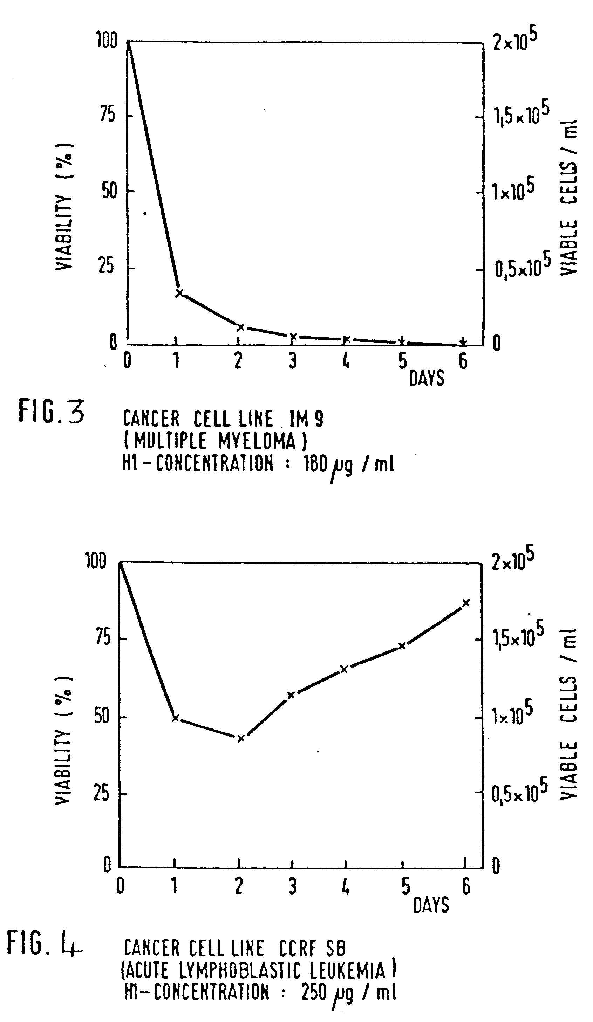

IM9 cells were very sensitive and showed a survival rate below 10 % within 3 days. After 6 days no living cells were observed (Fig. 3).

### Example 4

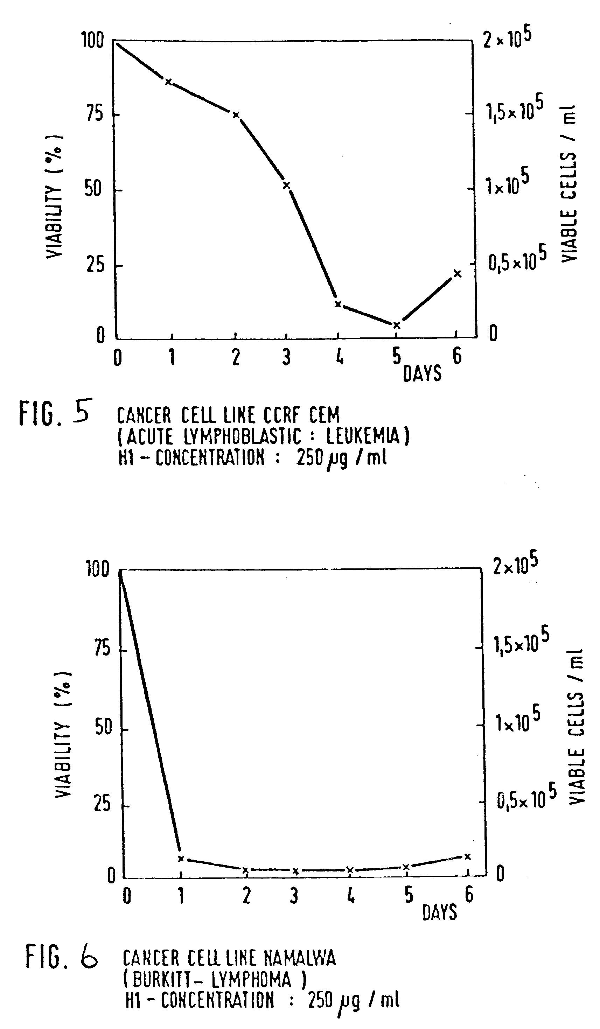

CCRF SB: A maximal death rate of 58 % of the cells was observed on the 3rd day. On the 6th day the cells grew up to 80 % of the initial cell concentration (Fig. 4).

### Example 5:

CCRF CEM: On the 5th day about 95 % of the cells were killed and on the 6th day a sligth growth had occured to about 20 % of the initial cell conentration (Fig. 5)

### Example 6

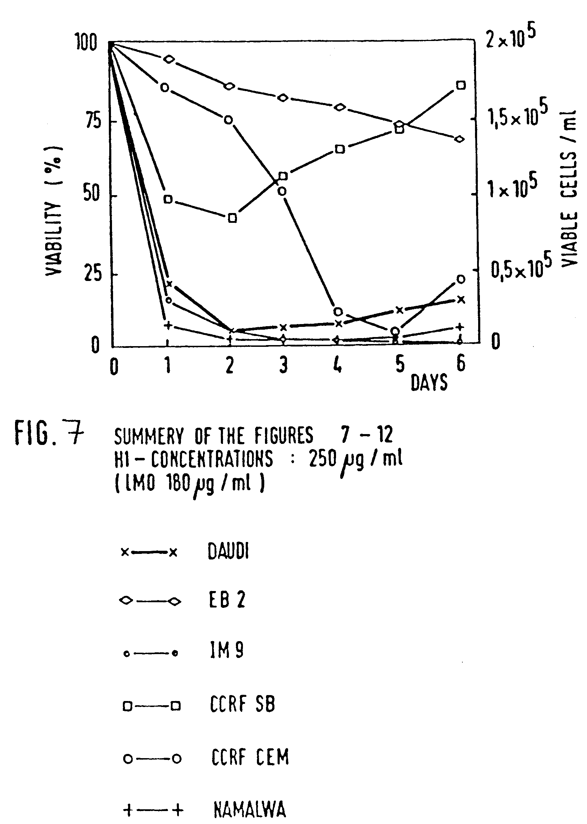

Namalwa was also very sensitive against H1 and showed a maximal decay rate of 99 % after 4 days. Little growth occured on day 5 and day 6 (2 % of the initial cell concentration) (Fig. 6)
To summarize, pure Histone H1 was cytoxic against all tested human cancer cells when it was applied as a single dose at a concentration of 180/250 mcg/ml. The cell line IM9 was the most sensitive one and was completely killed within 6 days. Three of the tested cell lines were Burkitt Lymphomas (Daudi, Namalwa & EB2). While more than 95% of the Daudi and Namalwa cells were killed, EB2 cells represent the most resistant cell line (death rate 30%). It is possible that this behaviour depends on the presence of EB-Virus particles. Both CCRF SB and CCRF CEM showed approximately 50% death rate on the 3^{rd} day. While the number of living cells of CCRF CEM was about 5% on day 5, the cells of CCRF SB were growing again from day 2 and reached about 80% of living cells in compared to the initial cell concentration (Fig. 7).

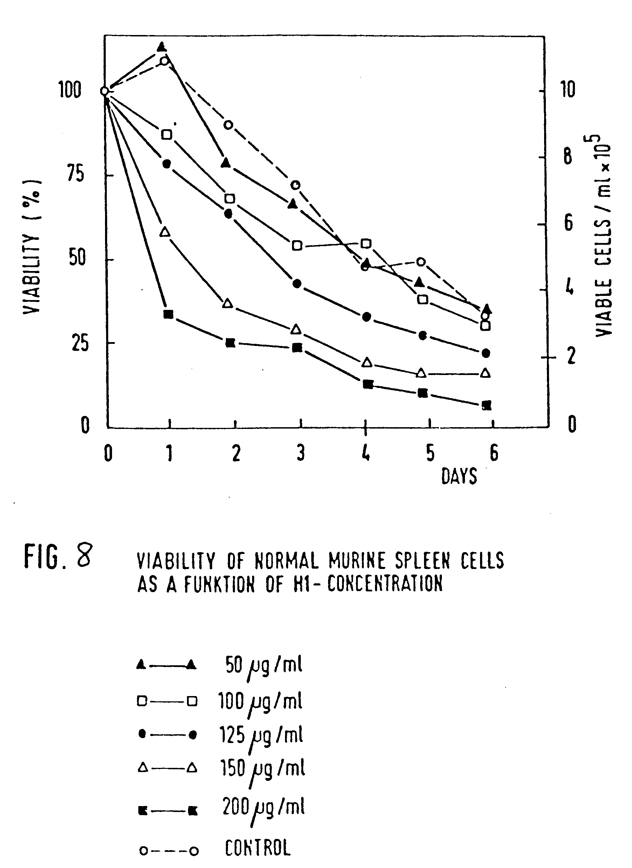

Fig. 8 shows the viability of normal murine spleen cells as function of H1-concentration from 50 µg/ml to 200 µg/ml.

### Example 7

### Concerning lymphoma:

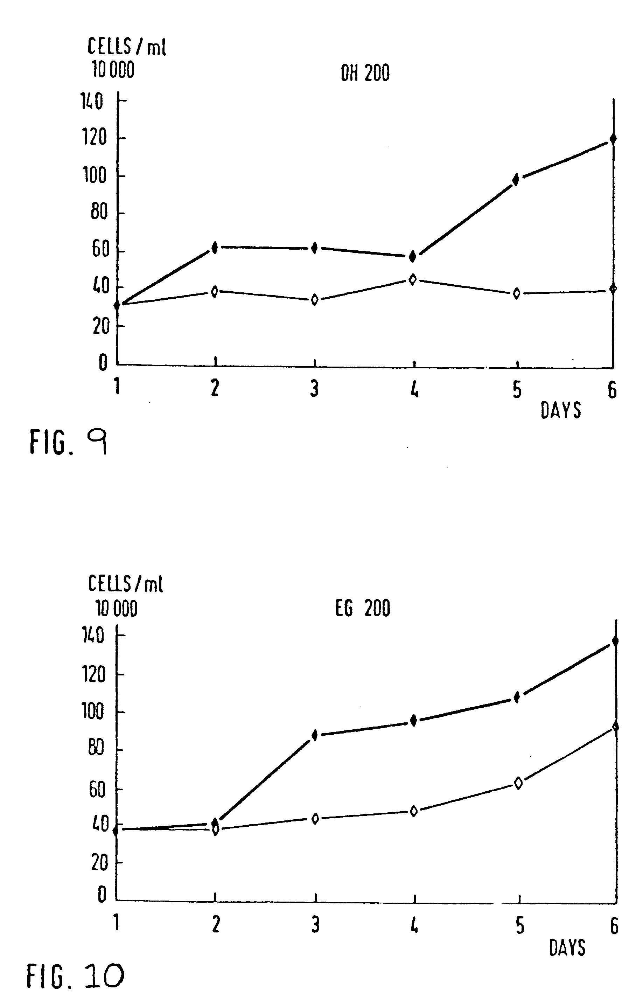

The cell line OH was obtained from a patient of the University Hospital of Lund, Department of Lund Medicine, and cultered as described in Example 1 execpt the cell density which was adjusted to 315 cell per se. Incubation with a single dose of 200 mg H1 Histone almost completely arrested the growth of the cells, whereas the control grew from 3 x 10⁵ cells perml to 1,2 x 10⁶ cells per ml within six days. (Fig. 9;(OH200))

### Example 8

### Concerning melanoma:

The cell line E6 was obtained from the University Hospital of Lund and cultivated as described in Example 1. The first day of incubation with a single dose of 200 mg per ml of histone H1 the cell density was determind to 40 x 10⁴ cells per ml. In the sample 1 treated with histone H1 the cell number stayed roughly constant until day four and increased to 90 x 10⁴ cells per ml at day 6. In the control the number of cells increased to 90 x 10⁴ cells per ml at day 3 and to 140 x 10⁴ cells per ml at day 6 (Fig. 10; (EG 200)).

### Examples 9

### Concerning sarcoma:

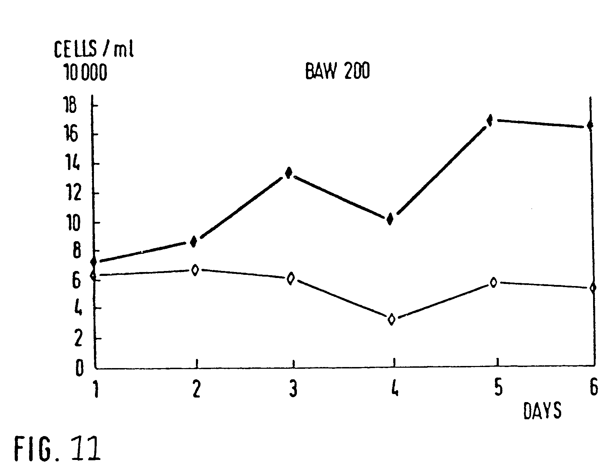

The cell line BAW was obtained from a patient of the University Hospital at Lund and cultivated as described in Example 1. The cell density was determind to 6 x 10⁴ cells per ml in the experiment and to 7 x 10⁴ viable cells per ml in the control immediately upon incubation with 200 mg per ml histone H1. As shown in Fig. 11 (BAW 200) after day 5 the number of viable cells had decreased below 5 x 10⁴ cells per ml whereas the untreated control had increased to about 17 x 10⁴ cells per ml.

## Claims

1. Pure histone H1 including all subtyps or active segments thereof with hormone or hormone like activity especially thymic hormone like activity for use in the therapeutic procedures.

2. A histone according to claim 1 for the immunotherapy, the therapy of endocrine malfunctions and cancer therapy.

3. A histone according to claim 1 for the treatment of the consequences of the thymectomy or the following massive irradiation of the thymus.

4. A histone according claim 1 for the treatment of radiation-induced leukemia or carcinoma.

5. A histone according to claim 1 for suppressing the activity of the thyroxine or ACTH or desoxycorticosterone or thyrotropin or gonadotropin.

6. A histone according to claim 1 for the treatment with the purpose of the strengthing the activity of the groth hormone.

7. A histone according to claim 2 for the treatment of carcinoma melanoma, sarcoma, mesothelioma and malignant diseases, especially, those of the lymphatic system originating from malignant B- and T-cells such as B-lymphoblastic lymphoma, myelogenic, leukemia, Burkitt-lymphoma.

8. A histon according to claim 1, which is extracted from an endocrine gland of an animal especially from the thymus gland of a calf.

9. A histone according to one of the claims 1 to 8 wherein the pure histone H1 is in connection with at least one protein of the immune system.

10. A histone according to one of the claims 1 to 8 wherein the pure histone H1 is in connection with ubiquitin.

## Patentansprüche

1. Reines Histon H1 einschließlich aller ihrer Untertypen oder ihrer aktiven Segmente mit hormoneller, insbesondere thymushormonartiger Wirksamkeit für therapeutische Zwecke.

2. Histon nach Anspruch 1 zur Immuntherapie, zur Therapie von endokrinen Störungen und zur Krebstherapie.

3. Histon nach Anspruch 1 zur Behandlung der Folgen einer Thymectomie oder massiver Bestrahlung des Thymus.

4. Histon nach Anspruch 1 zur Behandlung von strahlungs-induzierter Leukämie oder strahlen-induzierter Krebserkrankung.

5. Histon nach Anspruch 1 zur Unterdrückung der Wirksamkeit des Thyroxins oder des ACTH oder des Desoxycorticosterons oder des Thyrotropins oder des Gonadotropins.

6. Histon nach Anspruch 1 zur Behandlung mit dem Ziel der Stärkung der Wirkung des Wachstumshormons.

7. Histon nach Anspruch 2 zur Behandlung von Karzinomen, Melanomen, Sarcomen, Mesotheliomen und malignen Erkrankungen, insbesondere solchen des lymphatischen Systems verursacht durch maligne B- und T-Zellen, wie B-lymphoblastisches Lymphom, myelogenische Leukämie, Burkitt-Lymphom.

8. Histon nach Anspruch 1 das von tierischer endocriner Drüse, insbesondere von der Kalbsthymusdrüse extrahiert ist.

9. Histon nach einem der Ansprüche 1 bis 8, wobei reines Histon H1 mit wenigstens einem Protein des Immunsystems verbunden ist.

10. Histon nach einem der Ansprüche 1 bis 8, wobei reines Histon H1 mit Ubiquitin verbunden ist.

## Revendications

1. Histone pure H1, y compris tous ses sous-types ou ses segments actifs présentant une activité hormonale ou de type hormonale, plus particulièrement une activité hormonale de type thymique, utilisable dans des procédés thérapeutiques.

2. Histone selon la revendication 1 pour l'immunothérapie, le traitement des troubles endocriniens et le traitement anti-cancéreux.

3. Histone selon la revendication 1 pour le traitement des conséquences de la thymectomie ou d'une irradiation massive du thymus.

4. Histone selon la revendication 1 pour le traitement de la leucémie ou du cancer induits par radiation.

5. Histone selon la revendication 1 pour supprimer l'activité de la thyroxine ou de l'ACTH ou de la déxocycorticostérone ou de la thyrotropine ou de la gonadotrophine.

6. Histone selon la revendication 1 pour un traitement visant à renforcer l'activité de l'hormone de croissance.

7. Histone selon la revendication 2 pour le traitement du cancer, du mélanome, du sarcome, du mésothéliome, et des maladies malignes, en particulier celles du système lymphatique dues aux lymphocytes B et T malins comme le lymphome B-lymphoblastique,la leucémie myélogène, le lymphome de Burkitt.

8. Histone selon la revendication 1, extraite de la glande endocrine d'un animal plus particulièrement du thymus d'un veau.

9. Histone selon l'une quelconque des revendications 1 à 8 dans laquelle l'histone pure H1 est en liaison avec au moins une protéine du système immunitaire.

10. Histone selon l'une quelconque des revendications 1 à 8 dans laquelle l'histone pure H1 est en liaison avec l'ubiquitine.
